# EUROPEAN PATENT SPECIFICATION

(11) **EP 0 821 923 B1**
(45) Date of publication and mention of the grant of the patent: **05.12.2001**
(21) Application number: 97111443.4
(22) Date of filing: 07.07.1997
(51) Int. Cl.: A61F 2/36

(54) **A not cemented femoral stem for a hip prosthesis**
Zementfreier Femurschaft für Hüftprothese
Tige fémorale non cimentée pour prothése de hanche

(30) Priority: 08.07.1996 IT VI960112
(43) Date of publication of application: 04.02.1998
(73) Proprietor: CASTAMAN, Enrico, I-36100 Vicenza (IT)
(72) Inventor: CASTAMAN, Enrico, I-36100 Vicenza (IT)
(74) Representative: Bonini, Ercole

(56) References cited:
- EP-A- 0 145 939
- EP-A- 0 159 462
- EP-A- 0 222 236
- FR-A- 2 637 494

## Description

The invention concerns a not cemented femoral stem for hip prosthesis.

As it is well known, in surgical operations for the substitution of the femoral head in patients affected by serious hip pathology, suitable femoral stems are used which consists of metallic elements realized in stainless steel and titanium alloy which are inserted in the straight body of the femoral bone. Further, such stems are provided with a lateral body on which a spherical element is applied which is internally coupled with a cap placed in the hip.

The central body of the stem which is inserted in the straight body of the femur, is provided with a plurality of finnings which go longitudinally along it and which have a double function and precisely:
- at the opening phase of the implant, they perform a temporary anchorage capable of avoid the yielding in short time by the prompt mechanical fixing obtaining a strict and rigid contact between the bone and the inserted element;
- at the subsequent phase of the implant, they realize a secondary support or definite bone integration with progressive biological fixing due to the incorporation of the insert in the bone by an osteogenetic process of superficial holding which prevents the possible loosening with the time.

However, the femoral stems of the known technique which have now replaced the cemented prosthesis, present some disadvantages. A first disadvantage is that the longitudinal finnings available on the main body do not go along its whole length but are generally limited in the medium-upper part. This entails not homogeneously distributed strains on all the prosthesis surface and a less control on the coupling as in some parts there is not contact among prosthesis and bone.

A further disadvantage is that the longitudinal finnings of the main body are joined together to the main body by concave profiles formed by consecutive segments which realize broken lines. Such profiles do not guarantee an uniform growth of the bone as the efforts which are generated in correspondence of the comers are not uniformly distributed and so the continuity of the bone growth is not guaranteed; as it is known, the growth is stimulated only if a continuous contact between bone and prosthesis is permitted.

*The EP-A-0159462 discloses a femoral stem comprising a curved upper* *section and a conically tapering lower section with a plurality of projections extending longitudinally and parallel one each other. Said kind of projections do not assure a strong adhesion of the stem to the bone.*

The present invention proposes to overcome such disadvantages.

In particular it is one of the purposes of the invention to realize a not cemented femoral stem for hip prosthesis which, compared to the equivalent femoral stems of the known technique, realizes a better adherence bone-metal.

It is another purpose of the invention that the adherence bone-metal realized by said femoral stem is constant with the time.

It is not last purpose of the invention that said adherence bone-metal, realized by the femoral stem object of the invention, is homogeneously distributed.

Said purposes are achieved with the realization of a femoral stem for hip prosthesis which according to the main claim comprises:
- a central body having a mainly longitudinal extent and a substantially conical and longitudinal profile, suitable for being axially inserted in an excavation realized in the straight body of the femoral bone;
- a lateral body realized in one body with said central body and placed sideways at least one longitudinal midplane of said central body in correspondence of its largest cross-section;
- an appendix realized at the extremity of said lateral body, suited to receive a spherical element which is coupled with a cap placed in the hip having an inner profile conjugate to said spherical element, said central body being provided with a plurality of longitudinal finnings placed outwardly, substantially along its whole length, said finnings being joined at the base by curved surfaces having a transversal concave profile in order to define, outwardly said main body, a plurality of longitudinal grooves having a concave-curve bottom, characterised in that said finnings are convergent towards the extremity of the central body itself located in the opposite side of said lateral body.

Said purposes will be better stressed during the description of a preferred embodiment of the invention, given as an example but not as a restriction and represented in the enclosed drawings wherein:
- fig. 1 shows a side view of the femoral stem of the invention;
- fig. 2 shows a front view of the femoral stem of fig. 1;
- figs. 3, 4, 5 and 6 show three cross-sections of the central body of the femoral stem of the invention, realized in three different positions, orthogonal to the longitudinal axis of the same central body;
- fig. 7 shows the section of fig. 3, in which the central body of the femoral stem is inserted in a femoral bone.

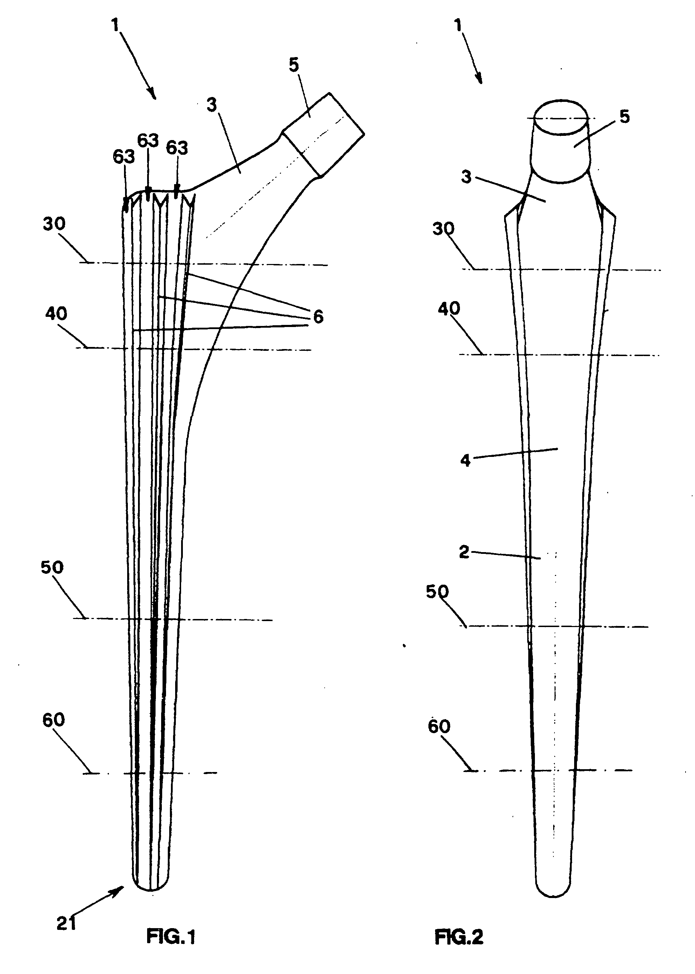

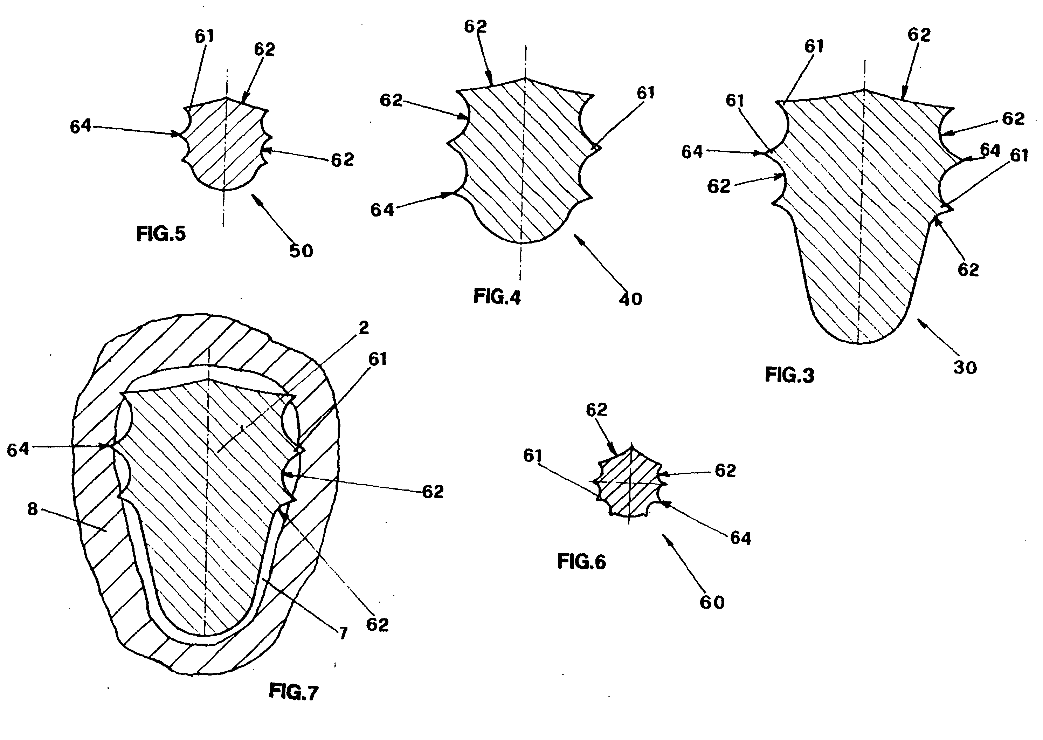

As it can be observed in figs. 1 and 2, the femoral stem of the invention, indicated as a whole with 1, comprises:
- a central body 2 having a mainly longitudinal extent and a substantially conic longitudinal profile, suitable for being inserted in the straight body of the femoral bone 8 visible in fig. 7;
- a lateral body 3 consisting of a one body with said central body 2, placed sideways at least one longitudinal midplane 4 of said central body 2 and in correspondence of its largest cross-section;
- an appendix 5 realized at the extremity of said lateral body 3, suited to receive a spherical element, not represented, which is coupled with a cap, not represented, placed in the hip and having an inner profile conjugate with said spherical element.

It can be observed that said central body 2 is provided with a plurality of longitudinal finnings 6 each of them presenting a substantially triangular profile 61 with the vertex 64 sharp-edged that aids the adherence and bone penetration, as it can be observed in the cross-sections of the central body 2 represented in figs. 3, 4, 5 and 6 and realized according to the cross-sections 30, 40, 50 and 60.

Besides, said longitudinal finnings 6, as it can be observed, are distributed in an equal number on both sides of the longitudinal midplane 4. On each transversal section 30, 40, 50 and 60, said substantially triangular profiles 61 are connected at the base by a curve surface 62 with a transversal concave outline, in order to define, externally to said main body 2, a plurality of longitudinal grooves 63 convergent towards the tip 21 of the central body 2 positioned on the opposite side of said lateral body 3.

Besides, said longitudinal grooves 63 and said longitudinal finnings 6 go along the central body 2 of the femoral stem 1 substantially on its whole length as it can be observed in fig. 1.

The femoral stem of the invention realises a greater adherence bone-metal compared to the equivalent not cemented femoral stems, because the maximum adherence bone-metal depends on two factors and precisely:
- the similarity of the central body 2 of the femoral stem to the natural endomedullar extension that consists of a pyramidal void with the shape of a truncated cone having a trapezoidal base in the epiphysis and that corresponds substantially to the section represented in fig. 3 and in fig. 7; the similarity to the void with the shape of a truncated cone having an elliptical base in the metaphysis and that corresponds to the section represented in fig. 4; the similarity to a truncated cone having a circular base in the diaphysis as it can be observed in the section represented in fig. 6;
- the finishing of the natural endomedullar extension by an excavation 7 visible in fig. 7, which reproduces as negative, the shape of the cross-section of the central body 2 of the femoral stem in every section according to the longitudinal direction.

Therefore, the central body 2 realises the maximum adherence bone-metal because, as it has been said and as it can be observed in the drawings, consists of a conic tap-root mean having a cross-section variable from a trapezoidal shape to an intermediate elliptic shape and a final rounded one.

Considering the constant adherence bone-metal with the time, this is derived from the embedding possibility of the central body 2 into the void extension of the bone, in relation to the burden and in presence of mechanical stresses.

The conicity of the central body controls the adjustment of the stem into the bone, developing a fast endomedullar seizure immediately after the implant and at each time that loosening is produced due to biological variation of the wall. In this way it is obtained an automatic and standing preservation of the bone-metal contact with the only condition that the implant has to be functionally stimulated.

Further, a very good homogeneous adherence bone-metal can be obtained from the global regular distribution of the pressure of the bone-metal interface due to the presence of the longitudinal finnings 6, whose the transversal substantially triangular outline 61 having a sharp edge 64, is fixed in the bone 8 as it can be observed in fig. 7.

Such finnings 6 are means of variable pre-contact able to escape excessive pressure by a sinking in the bone 8 suited to restore a greater adherence to the back zones. This creates an adjustment process of the superficial forces that aids a homogenous stimulation for the growth of the bone, supported by the continuous state of the curved surfaces 62 with a concave transversal outline which, as it can be observed in the cross-sections, connects at the base the longitudinal finnings 6 defining, according to the longitudinal direction of the central body 2, a plurality of longitudinal grooves 63.

Besides, the decrease of the height of the triangular protrusions 61 of the longitudinal finnings 6 beginning from the cross-section 30 with a greater section till the bottom 21 of the central body 2 passing through the cross sections 40 and 50, corresponds to a deeper penetration of the proximal bone than the distal one, in order to sink and to be adherent to it in the most homogeneous way. In other words the peripheral finnings system is an absorber which allows, under a burden, the dynamic approaching of the prosthesis to the bone, absorbing the impacts with parietal sinking effects in the greater concentration points and gaining new contacts in the points relieved from the effort: in this way, the anatomic distribution of the superficial pressure and the spontaneous improvement of a homogeneous loading capacity are obtained.

All that has been said, is due to said longitudinal finnings going along the central body on its whole length and they, on any transversal plane, are connected at the base by curved surfaces 62 with a concave outline. This implies the achievement of all the prefixed purposes.

It is evident that the femoral stem of the invention can be made of any materials prescribed by the rules or of other possible materials that could be suitable or useful for the matter.

Besides, the number of longitudinal finnings could be whichever according to the different uses and applications.

However, additional different embodiments have to be considered protected by the present invention.

## Claims

1. Femoral stem (1) for hip prosthesis comprising:
a central body (2) having a mainly longitudinal extent and a substantially conic longitudinal profile suitable for being axially inserted in an excavation (7) realized in the straight body of the femoral bone (8);
a lateral body (3) realized in one body with said central body (2) and placed sideways at least one longitudinal midplane (4) of said central body (2) and in correspondence of its largest cross-section (30).
an appendix (5) realized at the extremity of said lateral body (3), suited to receive a spherical element which is coupled with a cap placed in the hip and having an inner profile conjugate with said spherical element,
said central body (2) being provided with a plurality of longitudinal finnings (6) placed outwardly, substantially along its whole length, said finnings (6) being joined at the base by curved surfaces (62) having a transversal concave profile in order to define, outwardly said central body (2), a plurality of longitudinal grooves (63) having a concave-curve bottom, **characterised in that** said finnings are convergent towards the extremity (21) of the central body (2) located in the opposite side of said lateral body (3).

2. Femoral stem (1) according to the claim 1) **characterized in that** said central body (2) presents at least one longitudinal midplane (4) suitable for dividing each of the cross-sections (30, 40, 50, 60) of said central body (2) in two symmetrical parts each of them presenting an equal number of longitudinal finnings (6).

3. Femoral stem (1) according to any of the claims 1) or 2) **characterized in that** each of said longitudinal finnings (6) presents a cross section having a triangular profile (61) whose vertex (64) is suitable for being inserted in said femoral bone (8).

4. Femoral stem (1) according to the claim 2) **characterized in that** said vertex 64 of said triangular profile (61) is sharp-edged.

## Patentansprüche

1. Femurschaft (1) für Hüftprothesen, folgendes umfassend:
- einen zentralen Körper (2) mit überwiegend länglicher Ausdehnung und einem im Wesentlichen kegelförmigen Längsprofil, dazu geeignet, axial in eine Aushöhlung (7) in dem geraden Körper des Oberschenkelknochens (8) eingefügt zu werden;
- einen mit dem zentralen Körper (2) aus einem Teil gewonnenen seitlichen Körper (3), der seitlich von wenigstens einer länglichen Zwischenebene (4) des zentralen Körpers (2) und in Entsprechung seines breitesten Querschnitts (30) positioniert ist;
- einen Anhang (5) am Ende des seitlichen Körpers (3), dazu geeignet, ein kugelförmiges Element aufzunehmen, das mit einer in der Hüfte platzierten Kappe verbunden ist und ein Innenprofil aufweist, das mit diesem kugelförmigen Element gepaart ist, wobei der zentrale Körper (2) im Wesentlichen über seine ganze Länge mit einer Vielzahl von nach außen gerichteten Längsrippen (6) versehen ist, die an der Basis durch gekrümmte Flächen (62) verbunden sind, die ein konkaves Querprofil aufweisen, um von dem zentralen Körper (2) nach außen eine Vielzahl von Längsrillen (63) mit konkav-gekrümmtem Boden zu definieren, **dadurch gekennzeichnet, dass** diese Rippen zum Ende (21) des zentralen Körpers (2) hin konvergieren, der im entgegengesetzten Ende des seitlichen Körpers positioniert ist.

2. Femurschaft (1) gemäß Patentanspruch 1), **dadurch gekennzeichnet, dass** der zentrale Körper (2) wenigstens eine längliche Zwischenebene (4) aufweist, die geeignet ist, jeden der Querschnitte (30, 40, 50, 60) des zentralen Körpers (2) in zwei symmetrische Teile zu teilen, von denen jeder die gleiche Anzahl Längsrippen (6) aufweist.

3. Femurschaft (1) gemäß Patentansprüchen 1) oder 2), **dadurch gekennzeichnet, dass** die Längsrippen (6) einen Querschnitt mit dreieckigem Profil (61) aufweisen, dessen Scheitelpunkt (64) geeignet ist, in den Oberschenkelknochen (8) eingesetzt zu werden.

4. Femurschaft (1) gemäß Patentanspruch 2), **dadurch gekennzeichnet, dass** der Scheitelpunkt (64) des dreieckigen Profils (61) scharfkantig ist.

## Revendications

1. Tige fémorale (1) pour prothèse de hance comprenant :
- un corps central (2) ayant une extension essentiellement longitudinale et un profil longitudinal essentiellement conique indiqué pour être inséré d'une manière axiale dans un point creux (7) réalisé dans le corps droit de l'os fémoral (8) ;
- un corps latéral (3) réalisé en un seul corps avec ledit corps central (2) et positionné de travers par rapport à au moins un plan intermédiaire longitudinal (4) dudit corps central (2) et au niveau de sa section transversale plus large (30);
- un appendice (5) réalisé sur l'extrémité dudit corps latéral (3), indiqué pour recevoir un élément sphérique qui est couplé avec un couvercle positionné sur la hance et ayant un profil interne conjugué avec ledit élément sphérique, ledit corps central (2) étant équipé d'une pluralité d'ailettes longitudinales (6) se trouvant dans la partie extérieure, essentiellement le long de toute sa longueur, lesdits ailerons (6) reliés à la base au moyen de surfaces courbes (62) ayant un profil transversal concave indiqué pour définir, dans la partie extérieure dudit corps central (2), une pluralité de rainures longitudinales (63) ayant un fond concave courbe, **caractérisée en ce que** lesdits ailerons convergent vers l'extrémité (21) du corps central (2) positionné dans la partie opposée dudit corps latéral (3).

2. Une tige fémorale (1 ) selon la revendication 1) **caractérisée en ce que** ledit corps central (2) présente au moins un plan intermédiaire longitudinal (4) indiqué pour diviser chacune des sections transversales (30, 40, 50, 60) dudit corps central (2) en deux parties symétriques chacune présentant un nombre égal d'ailerons longitudinaux (6).

3. Une tige fémorale (1) selon une quelconque des revendications 1) ou 2) **caractérisée en ce que** chacune desdits ailerons longitudinaux (6) présente une section transversale ayant un profil triangulaire (61) dont le sommet (64) est indiqué pour être inséré dans ledit os fémoral (8).

4. Une tige fémorale (1) selon la revendication 2) **caractérisée en ce que** le dit sommet (64) dudit profil triangulaire présente un bord coupant.
